# EUROPEAN PATENT SPECIFICATION

(11) **EP 1 915 171 B1**
(45) Date of publication and mention of the grant of the patent: **20.11.2013**
(21) Application number: 06789148.1
(22) Date of filing: 01.08.2006
(51) Int. Cl.: A61K 38/28, A61P 3/10

(54) **METHOD OF PRESERVING THE FUNCTION OF INSULIN-PRODUCING CELLS**
VERFAHREN ZUM ERHALT DER FUNKTION VON INSULIN-PRODUZIERENDEN ZELLEN
METHODE DE CONSERVATION DE LA FONCTION DES CELLULES PRODUISANT L'INSULINE

(30) Priority: 01.08.2005 US 704295 P
(43) Date of publication of application: 30.04.2008
(73) Proprietor: MannKind Corporation, Valencia, CA 91355 (US)
(72) Inventor: BOSS, Anders, Hasager, Princeton, NJ 08540 (US); CHEATHAM, Wayman, Wendell, Columbia, MD 21045 (US); DIAMOND, David, C., West Hills, CA 91304 (US)
(74) Representative: Potter Clarkson LLP
(86) International application number: PCT/US2006/030014
(87) International publication number: WO 2007/016600

(56) References cited:
- WO-A-2005/067964
- WO-A2-01/00654
- PFUETZNER A ET AL: "Influence of a small dose i.v..s.c and pulmonary insulin treatment on prandial glucose control in patients with type 2 diabetes (ABSTRACT 812)" PROGRAM AND ABSTRACTS OF THE ANNUAL MEETING OF THE EUROPEAN ASSOCIATION FOR THE STUDY OF DIABETES, XX, XX, 9 September 2001 (2001-09-09), pages 1-2, XP002329615

## Description

### FIELD OF THE INVENTION

The present invention relates to methods for decreasing pancreatic stress and to furthering the lifespan of insulin-producing cells in patients having insulin-related disorders in which there is inadequate early phase insulin release despite a capability to produce insulin.

### BACKGROUND OF THE INVENTION

Diabetes mellitus (hereinafter, diabetes) currently afflicts at least 200 million people worldwide. The two main sub-types of diabetes include types 1 and 2. Type 1 diabetes accounts for about 10% of the 200 million afflicted with diabetes. Type 1 diabetes is caused by autoimmune destruction of insulin-secreting β-cells in the pancreatic islets of Langerhans. Type 2 diabetes accounts for the remaining 90% of individuals afflicted, and the rate of prevalence is increasing. Type 2 diabetes is often, but not always, associated with obesity, and although previously termed late-onset or adult-onset diabetes, is now becoming increasingly more prevalent in younger individuals. Type 2 diabetes is caused by a combination of insulin resistance and inadequate insulin secretion.

### The Physiological Role of Insulin

In a non-stressed normal individual, the basal glucose level will tend to remain the same from day to day because of an intrinsic feedback loop. Any tendency for the plasma glucose concentration to increase is counterbalanced by an increase in insulin secretion and a suppression of glucagon secretion, which regulate hepatic glucose production (gluconeogenesis and release from glycogen stores) and tissue glucose uptake to keep the plasma glucose concentration constant. If the individual gains weight or becomes insulin resistant for any other reason, blood glucose levels will increase, resulting in increased insulin secretion to compensate for the insulin resistance. Therefore the glucose and insulin levels are modulated to minimize changes in these concentrations while relatively normal production and utilization of glucose are maintained.

Five different phases of insulin secretion have been identified: (1) basal insulin secretion wherein insulin is released in the postabsorptive state; (2) the cephalic phase wherein insulin secretion is triggered by the sight, smell and taste of food, before any nutrient is absorbed by the gut, mediated by pancreatic innervation; (3) first-phase insulin secretion wherein an initial burst of insulin is released within the first 5-10 minutes after the β-cell is exposed to a rapid increase in glucose, or other secretagogues; (4) second-phase insulin secretion wherein the insulin levels rise more gradually and are related to the degree and duration of the stimulus and (5) a third-phase of insulin secretion that has only been described *in vitro*. During these stages, insulin is secreted, like many other hormones, in a pulsatile fashion, resulting in oscillatory concentrations in the blood. Oscillations include rapid pulses (occurring every 8-15 minutes) superimposed on slower oscillations (occurring every 80-120 minutes) that are related to fluctuations in blood glucose concentration.

Insulin secretion can be induced by other energetic substrates besides glucose (particularly amino acids) as well as by hormones and drugs. Of note is that the insulin response observed after food ingestion cannot be accounted for solely by the increase in blood glucose levels, but also depends on other factors such as the presence of free fatty acids and other secretagogues in the meal, the neurally activated cephalic phase and gastrointestinal hormones.

When an individual is given an intravenous glucose challenge, a biphasic insulin response is seen which includes a rapid increase with a peak, an interpeak nadir and a subsequent slower increasing phase. This biphasic response is only seen when glucose concentration increases rapidly, such as after a glucose bolus or glucose infusion. A slower increase in glucose administration, what is seen under physiologic conditions, induces a more gradually increasing insulin secretion without the well-defined biphasic response seen in response to bolus infusion of glucose.

Modeling of first-phase insulin responses under normal physiologic conditions has demonstrated that, after a meal, glucose concentration increases more gradually (Cₘₐₓ reached in approximately 20 minutes) than seen with intravenous bolus injections of glucose (Cₘₐₓ reached in approximately 3-10 minutes).

Healthy pancreatic β-cells generate an early response to a meal-like glucose exposure that rapidly elevates serum insulin both in the portal circulation and in the periphery. Conversely, defective β-cells, which have an impaired first-phase insulin response, generate a sluggish response to the meal-like glucose exposure.

Increasingly, evidence indicates that an early relatively rapid insulin response following glucose ingestion plays a critical role in the maintenance of postprandial glucose homeostasis. An early surge in insulin concentration can limit initial glucose excursions, mainly through the inhibition of endogenous glucose production. Therefore the induction of a rapid insulin response in a diabetic individual is expected to produce improved blood glucose homeostasis.

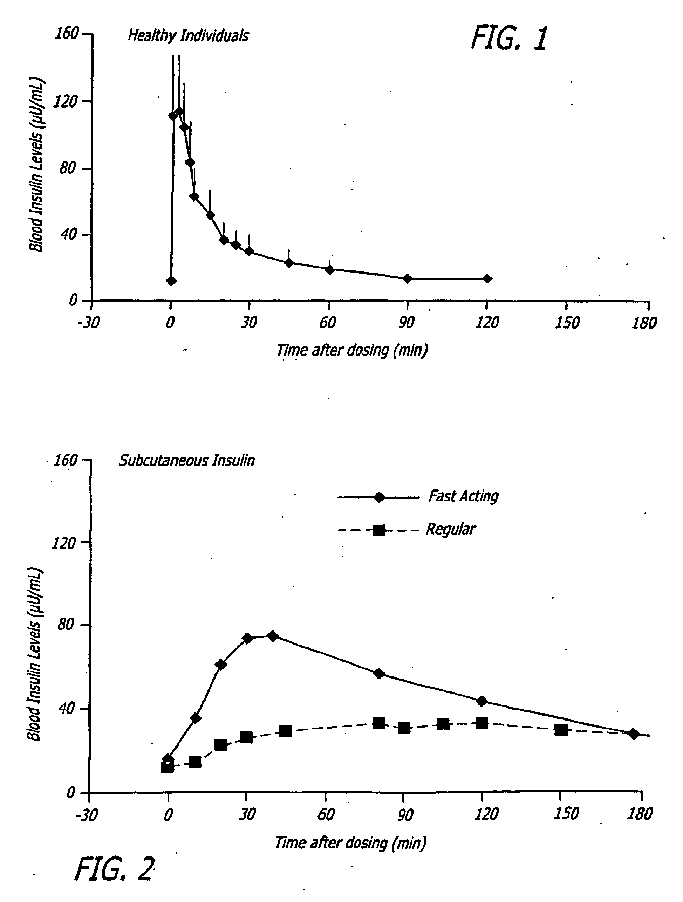

In a normal individual, a meal induces the secretion of a burst of insulin, generating a relatively rapid spike in serum insulin concentration that then decays relatively quickly (see Figure 1). This early-phase insulin response is responsible for the shut-off, or reduction, of glucose release from the liver. Homeostatic mechanisms then match insulin secretion (and serum insulin levels) to the glucose load. This is observed as a slow decay of modestly elevated serum insulin levels back to baseline and is second-phase kinetics.

### Diabetes

A central characteristic of diabetes is impaired β-cell function. One abnormality that occurs early in the disease progression in both type 1 and 2 diabetes is the loss of eating-induced rapid insulin response. Consequently, the liver continues to produce glucose, which adds to the glucose that is ingested and absorbed from the basic components of a meal.

Type 2 diabetics typically exhibit a delayed response to increases in blood glucose levels. While normal individuals usually begin to release insulin within 2-3 minutes following the consumption of food, type 2 diabetics may not secrete endogenous insulin until blood glucose begins to rise, and then with second-phase kinetics, that is a slow rise to an extended plateau in concentration. As a result, endogenous glucose production is not shut off and continues after consumption and the patient experiences hyperglycemia (elevated blood glucose levels). Another characteristic of type 2 diabetes is impaired insulin action, termed insulin resistance. Insulin resistance manifests itself as both a reduced maximal glucose elimination rate (GERmax) and an increased insulin concentration required to attain GERmax. Thus, to handle a given glucose load more insulin is required and that increased insulin concentration must be maintained for a longer period of time. Consequently, the diabetic patient is also exposed to elevated glucose concentrations for prolonged periods of time, which further exacerbates insulin resistance. Additionally, prolonged elevated blood glucose levels are themselves toxic to β cells.

Type 1 diabetes occurs as a result of the destruction of the insulin-producing cells of the pancreas (β-cells) by the body's own immune system. This ultimately results in a complete insulin hormone deficiency. However, during the period immediately following onset, most patients go through a "honeymoon" phase. While early phase insulin release has been lost, the remaining β-cells still function and produce some insulin, which is released with second-phase kinetics. Since even partial β-cell function can be critical in avoiding many of the long term complications of diabetes, one focus of current diabetes research is the preservation of the function of these residual β-cells.

Type 2 diabetes arises from different and less well understood circumstances. The early loss of early phase insulin release, and consequent continual glucose release, contributes to elevated glucose concentrations. High glucose levels promote insulin resistance, and insulin resistance generates prolonged elevations of serum glucose concentration. This situation can lead to a self-amplifying cycle in which ever greater concentrations of insulin are less effective at controlling blood glucose levels. Moreover, as noted above, elevated glucose levels are toxic to the β-cells, reducing the number of functional β-cells. Genetic defects impairing the growth or maintenance of the microvasculature nourishing the islets can also play a role in their deterioration (Clee, S.M., et al. Nature Genetics 38:688-693, 2006) Eventually, the pancreas becomes overwhelmed, and individuals progress to develop insulin deficiency similar to people with type 1 diabetes.

### Therapy

Insulin therapy is the standard treatment for type 1 diabetes, since few patients are identified in the honeymoon phase. While incipient type 2 diabetes can be treated with diet and exercise, most early stage type 2 diabetics are currently treated with oral antidiabetic agents, but with limited success. Patients generally transition to insulin therapy as the disease progresses. These treatments, however, do not represent a cure.

Current insulin therapy modalities can supplement or replace endogenously-produced insulin to provide basal and second-phase-like profiles but do not mimic first-phase kinetics (see Figure 2). Additionally, conventional insulin therapy often involves only one or two daily injections of insulin. However, more intensive therapy such as three or more administrations a day, providing better control of blood glucose levels, are clearly beneficial (see for example Nathan, D.M., et al., N Engl J Med 353:2643-53, 2005), but many patients are reluctant to accept the additional injections.

Until recently, subcutaneous (SC) injection has been the only route of delivering insulin to patients with both type 1 and type 2 diabetes. However, SC insulin administration does not lead to optimal pharmacodynamics for the administered insulin. Absorption into the blood (even with rapid acting insulin analogues) does not mimic the prandial physiologic insulin secretion pattern of a rapid spike in serum insulin concentration. Subcutaneous injections are also rarely ideal in providing insulin to type 2 diabetics and may actually worsen insulin action because of delayed, variable and shallow onset of action. It has been shown, however, that if insulin is administered intravenously with a meal, early stage type 2 diabetics experience the shutdown of hepatic glucose release and exhibit increased physiologic glucose control. In addition their free fatty acids levels fall at a faster rate that without insulin therapy. While possibly effective in treating type 2 diabetes, intravenous administration of insulin, is not a reasonable solution, as it is not safe or feasible for patients to intravenously administer insulin at every meal.

Despite improving progress in diabetes management, diabetes continues to be a disabling chronic condition, which if left untreated, may be associated with end-stage organ complications and premature death. Therefore, many researchers have looked to transplant approaches hoping that these would alleviate the need for chronic insulin injections, frequent blood glucose monitoring, and strict attention to diet and exercise. Recent advances in gene and cell-based therapies have provided hope for finding a cure for diabetes. These include efforts to regenerate existing β-cells by replication or neogenesis, manipulating embryonic stem cells to differentiate into β-cells, and utilizing pancreatic or liver precursor cells to serve as a source of insulin. Recent work has produced evidence of successful insulin production from transplanted genetically engineered liver cells from the diabetic patient's own liver. The most advanced approach to cellular therapy for diabetes, having reached clinical application, is the transplant of β-cells. In transplant patients, there is evidence of insulin independence in some of these treated individuals. β-cell transplantation is a less invasive than whole organ transplantation since only the endocrine portions of the pancreas (the islets) are transplanted via a percutaneous catheter. Results in this area of study were discouraging until advances made by Dr. James Shapiro of Edmonton, Canada (Shapiro et al., Diabetes July 2002, 5:2148). Dr. Shapiro developed The Edmonton Protocol. The Edmonton Protocol utilizes a corticosteroidfree anti-rejection regimen and the transplantation of a sufficient number of islets (requiring around 2-4 donor organs per transplant). The group has reported 7 consecutive patients with Type 1 diabetes were rendered insulin independent for 1 year following islet transplant. (Hirschberg B et al., Diabetes/Metabolism Research and Reviews 2003;19:175-178). However, beyond 3 years, the transplanted islets fail as indicated by a return to insulin therapy.

The premise behind islet transplantation is to process the organ donor's pancreas so as to isolate the 5% of the gland responsible for endocrine hormone secretion (the pancreatic Islets of Langerhans, or the β-cells thereof) away from the remaining 95% of the gland responsible for its exocrine functions (secretion of digestive enzymes). Once isolated, the insulin-producing islets are infused through a thin tube placed in the hepatic portal vein, which is the main vein that transports blood from the intestines to the liver. Once infused, the bloodstream transports the islets into the liver where they lodge and begin making insulin to regulate blood sugar. Current procedures utilize about 2-4 donor pancreases (from cadavers) and a corticosteroid free immunosuppressive therapy regimen to prevent transplant rejection.

One problem with the islet transplant procedure is the longevity of the islet cells. Patients are insulin independent for as long as 2 years. Thereafter, they generally return to insulin therapy, although at lower dosage(s) than pre-transplant. The reason for islet failure is not clear, but it has been suggested that the islet cells are stressed and overall function is compromised.

Glycemic control achieved in islet transplant recipients is usually superior to that achieved with Insulin treatment, and diet and exercise. As long as the islet function persists, there have been few, if any, reports of severe hypoglycemia episodes. However, patients do not regain 'normal' counterregulatory hormone responses to hypoglycemia. Only a small number of patients achieve normal blood glucose levels according the American Diabetes Association criteria. When provoking an acute first-phase insulin response via intravenous glucose infusion, islets of successfully transplanted patients show a markedly diminished insulin peak compared to normal individuals.

Thus, type 1 diabetics in the "honeymoon" phase of the disease, type 2 diabetics (with remaining β-cell function), and β-cell transplant recipients despite differing etiologies, all have the following similar deficit in pancreatic function: inadequate early phase Insulin release and second-phase release of diminished effectiveness. It is an object of the present invention to compensate for the lack of the physiologic early phase release and thereby prolong or preserve β-cell function.

### SUMMARY OF THE INVENTION

A first aspect of the invention provides an insulin composition for use in the preservation of insulin-producing cells in a patient with type 1 diabetes in the honeymoon phase or in a patient with type 1 diabetes who is a recipient of an insulin-producing cell transplant wherein at least one dose of said composition is administered prandially to said patient and wherein said composition mimics a physiological meal-related early phase insulin response, such that serum insulin levels peak within 15 minutes of administration, and preserves the function of said insulin-producing cells.

A second aspect of the invention provide a use of an insulin composition in the manufacture of a medicament for the preservation of insulin-producing cells In a patient with type 1 diabetes in the honeymoon phase or in a patient with type 1 diabetes who is a recipient of an insulin-producing cell transplant wherein at least one dose of said composition is administered prandially to said patient and wherein said composition mimics a physiological meal-related early phase insulin response, such that serum insulin levels peak within 15 minutes of administration, and preserves the function of said insulin-producing cells.

Compositions useful for decreasing pancreatic stress and furthering the lifespan of insulin-producing cells in type 1 diabetics in the honeymoon phase, and islet transplant patients are provided. Embodiments of the composition are for use in mimicking the meal-related early phase insulin response, using a dose sufficient to reduce serum levels of proinsulin and/or to control glucose excursions. Mimicking early phase kinetics, peak serum insulin levels can be reached within about 12 to within about 30 minutes of administration. Serum insulin levels can also return to baseline within about two or three hours of administration. Also disclosed herein, Insulin is administered to a patient in need of insulin therapy at mealtime, that is, within about 10 minutes, preferably 5 minutes before, or 30, 25, 15, or 10 minutes after starting a meal. (The shorter times after being preferred for patients with normal gastric emptying, the longer times after being appropriate for patients with delayed gastric emptying) In a preferred embodiment, a pulmonary delivery is achieved by inhalation of a dry powder formulation of a fumaryl diketopiperazine complexed with insulin facilitated by use of a unit dose inhaler. The term "fumaryl diketopiperazine" (FDKP) as used herein also includes the salts thereof. Preferred dosages are in the range of about 15 to 90 IU, or greater than 24 IU of insulin complexed with fumaryl diketopiperazine, or the equivalent.

Embodiments of the composition for use in increasing the lifespan of insulin producing cells include ones wherein pancreatic stress is measured as a loss of physiologic [or endogenous] first or early phase insulin response; wherein pancreatic stress is measured as an increase in serum proinsulin levels without adjunct insulin therapy; wherein oxidative stress due to acute glucose excursions is measured (e.g., as the 24-hour secretion rate of free 8-iso prostaglandin F_{2α} (8-isoPGF_{2α})) as a surrogate for pancreatic stress; and wherein pancreatic stress is determined by deterioration in the ability to control blood glucose levels in the absence of other treatment, reduction in secretory capacity (e.g., stimulated C-peptide), reduction in insulin sensitivity (e.g., HOMA-S: Homeostasis Model Assessment of Insulin Sensitivity). Longevity of insulin producing cells can also be assessed through measurements of β-cell mass or sensitivity to apoptosis.

Also disclosed is a composition useful for the preservation of insulin producing cells. The composition comprises a controlled-release insulin formulation or a delayed onset preparation including an insulin formulation.

A method is provided for preserving the function of insulin-producing cells in a non-insulin dependent patient having an insulin-related disorder, comprising: providing said non-insulin dependent patient having an insulin-related disorder, with an insulin dose; administering said insulin dose to said patient; wherein said insulin dose mimics a physiological meal-related early phase insulin response and preserves the function of said insulin-producing cells in said patient.

It is disclosed that the non-insulin dependent patient having an insulin-related disorder may be a type 1 diabetic in the honeymoon phase or may be an insulin-producing cell transplant recipient. Also disclosed herein, the non-insulin dependent patient having an insulin-related disorder is a type 2 diabetic.

In another embodiment of the present invention, the insulin dose is administered orally. In another embodiment, the insulin dose is inhaled. In yet another embodiment, the insulin dose comprises a dry powder formulation.

In an embodiment of the present invention, the insulin dose comprises a dose sufficient to reduce serum levels of proinsulin. In another embodiment, the insulin dose comprises a dose sufficient to control glucose excursions. In another embodiment, the insulin reaches peak serum levels within about 15 minutes of administration. In another embodiment, the peak serum insulin level is at least 60 mU/L. In another embodiment, the insulin dose is sufficient to control blood glucose levels. In yet another embodiment, the insulin dose is sufficient to reduce glucose release from the liver.

In another embodiment of the present invention, said insulin dose comprises a fumaryl diketopiperazine (FDKP) associated with insulin. In another embodiment, the insulin dose is within the range equivalent to about 15 IU to about 90 IU of FDKP insulin.

Also disclosed is a method for lessening post-prandial pancreatic stress in a non-insulin dependent patient having an insulin-related disorder comprising providing the non-insulin dependent patient having an insulin-related disorder to be treated, administering an insulin dose to the patient sufficient to control blood glucose levels and reduce serum levels of proinsulin; and wherein the insulin dose mimics the physiologic meal-related early phase insulin response and pancreatic stress is attenuated.

Also disclosed is a method for increasing longevity of an insulin-producing cell transplant in a patient, comprising providing an insulin-producing cell transplant recipient to be treated, administering an insulin dose to the patient sufficient to control blood glucose levels and reduce serum levels of proinsulin; and wherein the insulin dose mimics the physiologic meal-related early phase insulin response and pancreatic stress is attenuated and longevity of the insulin-producing cells is achieved.

Also disclosed is a method for preserving the function of insulin-producing cells in a patient, comprising, providing a non-insulin dependent patient having an insulin-related disorder, an insulin dose and an immunosuppressive medication; administering the insulin dose to the patient wherein the insulin dose mimics the physiologic meal-related early phase insulin response; and administering the immunosuppressive medication to the patient in conjunction with the insulin dose to slow an auto-immune response.

Also disclosed is a composition useful for the preservation of insulin-producing cells in a non-insulin dependent patient having an insulin-related disorder comprising a controlled-release insulin formulation.

Also disclosed is a composition useful for the preservation of insulin-producing cells in a non-insulin dependent patient having an insulin-related disorder comprising a delayed onset preparation including an insulin formulation.

Also disclosed is a method for preserving the function of insulin-producing cells in a non-insulin dependent patient having an insulin-related disorder, comprising: providing a non-insulin dependent patient having an insulin-related disorder, wherein said patient is a type 1 diabetic in the honeymoon phase or an insulin-producing cell transplant recipient, and an insulin dose; administering said insulin dose to said patient; and wherein said insulin dose mimics a physiological meal-related early phase insulin response and preserves the function of said insulin-producing cells in said patient.

Also disclosed is a method for preserving the function of insulin-producing cells in a patient having an insulin-related disorder, comprising providing a patient having an insulin-related disorder, wherein the patient has not been treated with an insulin composition other than basal insulin, with an insulin dose; administering the insulin dose to the patient; and wherein the insulin dose mimics a physiological meal-related early phase insulin response and preserves the function of the insulin-producing cells in the patient.

Also disclosed is a method for preserving the function of insulin-producing cells in a patient having an insulin-related disorder, comprising providing a patient having an insulin-related disorder, wherein the patient has lost early phase insulin release and has a level of serum glycated hemoglobin (HbA1 c) less than 8%, with an insulin dose, administering the insulin dose to the patient, and wherein the insulin dose mimics a physiological meal-related early phase insulin response and preserves the function of the insulin-producing cells in the patient. In another embodiment, the insulin dose is administered with any meal containing more than 15 g of carbohydrate. In another embodiment, the patient is not on a prandial insulin regimen. In another embodiment, the patient has serum proinsulin levels within a normal range. In another embodiment, the patient has elevated mean amplitude of glucose excursions. In yet another embodiment, the patient has evidence of elevated oxidative stress and the oxidative stress is measured by 8-iso PGF(2a) levels. In another embodiment, the level of serum HbA1c is less than 7%. In yet another embodiment, the level of serum HbA1c is less than 6.5%.

### BRIEF DESCRIPTION OF THE DRAWINGS

Figure 1 depicts the measurement of first-phase insulin release kinetics following artificial stimulation by bolus glucose infusion.

Figure 2 depicts serum insulin concentration after administration of subcutaneous (SC) regular human insulin or SC fast acting insulin (Novolog™). Novolog™ is a registered trademark of Novo Nordisk Pharmaceuticals, Bagsvaerd, Denmark.

Figure 3 depicts the glucose elimination rate after administration of TECHNOSPHERE^{®}/Insulin in humans according to the teachings of the present invention.

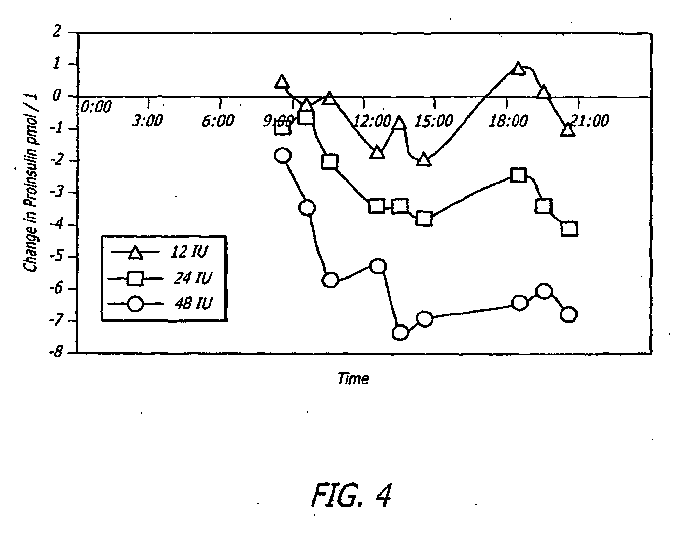

Figure 4 depicts the changes in proinsulin levels after administration of TECHNOSPHERE^{®}/Insulin in humans according to the teachings of the present invention.

### DEFINITION OF TERMS

Prior to setting forth the invention, it may be helpful to provide an understanding of certain terms that will be used hereinafter:

Dry powder: As used herein "dry powder" refers to a fine particulate composition that is not suspended or dissolved in a propellant, carrier, or other liquid. It is not meant to imply a complete absence of all water molecules.

Early phase: As used herein "early phase" refers to the rise in blood insulin concentration induced in response to a meal. This early rise in insulin in response to a meal is sometimes referred to as first-phase.

Excursion: As used herein, "excursion" refers to blood glucose concentrations that fall either above or below a pre-meal baseline or other starting point. Excursions are generally expressed as the area under the curve (AUC) of a plot of blood glucose over time. AUC can be expressed in a variety of ways. In some instances there will be both a fall below and rise above baseline creating a positive and negative area. Some calculations will subtract the negative AUC from the positive, while others will add their absolute values. The positive and negative AUCs can also be considered separately. More sophisticated statistical evaluations can also be used. In some instances it can also refer to blood glucose concentrations that rise or fall outside a normal range. A normal blood glucose concentration is usually between 70 and 110 mg/dL from a fasting individual, less than 120 mg/dL two hours after eating a meal, and less than 180 mg/dL after eating.

First-Phase: As used herein, "first-phase" refers to the spike in insulin levels as induced by a bolus intravenous injection of glucose. A first-phase insulin release generates a spike in blood insulin concentration that is a rapid peak which then decays relatively quickly. The first-phase insulin release is also referred to as early phase.

Glucose elimination rate: As used herein, "glucose elimination rate" is the rate at which glucose disappears from the blood and is determine by the amount of glucose infusion required to maintain stable blood glucose, often around 120 mg/dL during the study period. This glucose elimination rate is equal to the glucose infusion rate, abbreviated as GIR.

Honeymoon phase: As used herein, the "honeymoon phase" of type 1 diabetes refers to the early stages of the disease where early phase insulin release has been lost and the remaining β-cells still function and produce some insulin, which is released with second-phase kinetics.

Hyperglycemia: As used herein, "hyperglycemia" is a higher than normal fasting blood glucose concentration, usually 126 mg/dL or higher. In some studies hyperglycemic episodes were defined as blood glucose concentrations exceeding 280 mg/dL (15.6mM).

Hypoglycemia: As used herein, "hypoglycemia" is a lower than normal blood glucose concentration, usually less than 63 mg/dL 3.5 mM), Clinically relevant hypoglycemia is defined as blood glucose concentration below 63 mg/dL or causing patient symptoms such as hypotonia, flush and weakness that are recognized symptoms of hypoglycemia and that disappear with appropriate caloric intake. Severe hypoglycemia is defined as a hypoglycemic episode that required glucagon injections, glucose infusions, or help by another party.

In proximity: As used herein, "in proximity," as used in relation to a meal, refers to a period near in time to the beginning of a meal.

Insulin composition: As used herein, "insulin composition" refers to any form of insulin suitable for administration to a mammal and includes insulin isolated from mammals, recombinant insulin, insulin associated with other molecules and also includes insulin administered by any route including pulmonary, subcutaneous, nasal, oral, buccal and sublingual. Insulin compositions can be formulated as dry powders or aqueous solutions for inhalation; aqueous solutions for subcutaneous, sublingual, buccal, nasal or oral administration and solid dosage forms for oral and sublingual administration.

Insulin-related disorder: As used herein, "insulin-related disorders" refers to disorders involving production, regulation, metabolism, and action of insulin in a mammal. Insulin-related disorders include, but are not limited to, pre-diabetes, type 1 diabetes mellitus, type 2 diabetes mellitus, hypoglycemia, hyperglycemia, insulin resistance, secretory dysfunction, loss of pancreatic β-cell function, and loss of pancreatic β-cells.

Non-insulin dependent patients having insulin-related disorders: As used herein "non-insulin dependent patients having insulin-related disorders" refers to patients with disorders for which therapy with exogenously-provided insulin is not the current standard treatment upon diagnosis. Non-insulin dependent patients having insulin-related disorders which are not treated with exogenously-administered insulin include early type 2 diabetes, type 1 diabetes in the honeymoon phase, pre-diabetes and insulin-producing cell transplant recipients.

Insulin resistance: As used herein, the term "insulin resistance" refers to the inability of a patient's cells to use insulin properly. The pancreas responds to this problem at the cellular level by producing more insulin. Eventually, the pancreas cannot keep up with the body's need for insulin and excess glucose builds up in the bloodstream. Patients with insulin resistance often have high levels of blood glucose and high levels of insulin circulating in their blood at the same time.

Microparticles: As used herein, the term "microparticles" includes microcapsules having an outer shell composed of either a diketopiperazine alone or a combination of a diketopiperazine and one or more drugs. It also includes microspheres containing drug dispersed throughout the sphere; particles of irregular shape; and particles in which the drug is coated in the surface(s) of the particle or fills voids therein.

Periprandial: As used herein, "periprandial" refers to a period of time starting shortly before and ending shortly after the ingestion of a meal or snack.

Postprandial: As used herein, "postprandial" refers to a period of time after ingestion of a meal or snack. As used herein, late postprandial refers to a period of time 3, 4, or more hours after ingestion of a meal or snack.

Potentiation: Generally, potentiation refers to a condition or action that increases the effectiveness or activity of some agent over the level that the agent would otherwise attain. Similarly it may refer directly to the increased effect or activity. As used herein, "potentiation" particularly refers to the ability of elevated blood insulin concentrations to boost effectiveness of subsequent insulin levels to, for example, raise the glucose elimination rate.

Prandial: As used herein, "prandial" refers to a meal or a snack.

Pre-Diabetic: As used herein, the term "pre-diabetic" refers to a patient with impaired fasting glucose or impaired glucose tolerance, that is with a fasting blood glucose level between 100 mg/dL (5.5 mmol/L) and 126 mg/dL (7.0 mmol/L), or a 2 hour post-prandial blood glucose level between 146 mg/dL (7.9 mmol/L) and 200 mg/dL (11.1 mmol/L).

Second-Phase: As used herein, "second-phase" refers to the slow decay of modestly elevated blood insulin levels back to baseline after the first-phase has passed. Second-phase can also refer to the non-spiking release of insulin in response to elevated blood glucose levels.

TECHNOSPHERE^{®}/Insulin: As used herein, "TECHNOSPHERE^{®}/Insulin" or "TI" refers to an insulin composition comprising regular human insulin and TECHNOSPHERE^{®} microparticles, a drug delivery system. TECHNOSPHERE^{®} microparticles comprise a diketopiperazine, specifically 3,6-di(fumaryl-4-aminobutyl)-2,5-diketopiperazine (fumaryl diketopiperazine, FDKP). Specifically, TECHNOSPHERE^{®}/Insulin comprises a FDKP/human insulin composition.

As used herein, "diketopiperazine" or "DKP" includes diketopiperazines and salts, derivatives, analogs and modifications thereof falling within the scope of the general Formula 1, wherein the ring atoms E₁ and E₂ at positions 1 and 4 are either O or N and at least one of the side-chains R₁ and R₂ located at positions 3 and 6 respectively contains a carboxylic acid (carboxylate) group. Compounds according to Formula 1 include, without limitation, diketopiperazines, diketomorpholines and diketodioxanes and their substitution analogs.

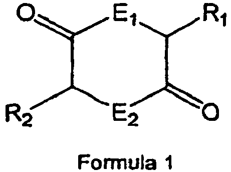

Diketopiperazines, in addition to making aerodynamically suitable microparticles, also facilitate transport across cell layers, further speeding absorption into the circulation. Diketopiperazines can be formed into particles that incorporate a drug or particles onto which a drug can be adsorbed. The combination of a drug and a diketopiperazine can impart improved drug stability. These particles can be administered by various routes of administration. As dry powders these particles can be delivered by inhalation to specific areas of the respiratory system, depending on particle size. Additionally, the particles can be made small enough for incorporation into an intravenous suspension dosage form. Oral delivery is also possible with the particles incorporated into a suspension, tablets or capsules. Diketopiperazines may also facilitate absorption of an associated drug.

In another embodiment of the present invention, the DKP is a derivative of 3,6-di(4-aminobutyl)-2,5-diketopiperazine, which can be formed by (thermal) condensation of the amino acid lysine. Exemplary derivatives include 3,6-di(succinyl-4-aminobutyl)-, 3,6-di(maleyl-4-aminobutyl)-, 3,6-di(glutaryl-4-aminobutyl)-, 3,6-di(malonyl-4-aminobutyl)-, 3,6-di(oxalyl-4-aminobutyl)-, and 3,6-di(fumaryl-4-aminobutyl)-2,5-diketopiperazine. The use of DKPs for drug delivery is known in the art (see for example U.S. Patent Nos. 5, 352,461, 5,503,852, 6,071,497, and 6,331,318". The use of DKP salts is described in co-pending U.S. Patent Application No. 11/210,710 filed August 23, 2005. Pulmonary drug delivery using DKP microparticles is disclosed in U.S. Patent No. 6,428,771.

TECHNOSPHERE^{®}/Placebo: As used herein, "TECHNOSPHERE^{®}/Placebo" refers to TECHNOSPHERE^{®} particles which are not associated with insulin.

Units of measure: Subcutaneous and intravenous insulin dosages are expressed in IU which is defined by a standardized biologic measurement. Amounts of insulin formulated with fumaryl diketopiperazine are also reported in IU as are measurements of insulin in the blood. TECHNOSPHERE^{®}/Insulin dosages are expressed in arbitrary units (U) which are numerically equivalent to the amount of insulin formulated in the dosage.

### DETAILED DESCRIPTION OF THE INVENTION

One aspect of the invention is to preserve β-cell function and thereby halt or attenuate the progression of diabetic disease. β-cell function is preserved by reducing the stress caused by the excessive demand for insulin that develops, through various mechanisms, in type 1 and 2 diabetes and following β-cell transplant procedures. Function can also be preserved by lessening glucose toxicity by reducing exposure to elevated glucose concentrations as herein described. Excessive insulin demand, poor control of blood glucose levels, and its consequent stresses on the β-cells, are associated with loss of function and β-cell death. Microvascular damage in the pancreas due to oxidative stress resultant from acute fluctuations in blood glucose concentrations can also play a role. As described herein, biosynthetic demand is reduced and stress is alleviated by non-intravenous administration of an insulin preparation that mimics physiologic mealtime early-phase insulin release.

As used herein, insulin-producing cells refers to β-cells of the Islets of Langerhans, liver cells or pancreatic precursor cells genetically engineered to produce insulin, embryonic or adult stem cells differentiated into β-cells, cells treated with an insulin gene therapy, or any cell type capable of producing and secreting insulin. Potential sources of β-cells for transplantation or regeneration in the pancreas were recently reviewed (Bonner-Weir, S. & Weir, G.C., Nature Biotech. 23:857-861, 2005). While aspects of the invention disclosed herein will predominantly be described as it applies to β-cells, it is to be understood that the methods and compositions so described can be similarly useful in preserving the functionality of any insulin producing cells subject to the stresses of excessive demand for insulin biosynthesis or glucose toxicity, etc. Thus, methods of mimicking early phase insulin response in type 1 diabetics in the honeymoon phase, early type 2 diabetics, and recipients of islet transplants or other insulin producing cells, are designed to increase the lifespan of the insulin producing cells by reducing pancreatic stress.

As used herein, mimicking physiologic mealtime early phase insulin release (or similar terms) does not necessarily indicate exact replication of all features of the physiologic response. It can refer to methodologies producing a spike or peak of insulin concentration in the blood that constitutes both a relatively quick rise (less than 30 minutes, preferably less than about 20 minutes or 15 minutes from administration or first departure from baseline) and fall (descent through half maximal by 80 minutes, preferably 50 minutes, more preferably 35 minutes after peak) in concentration. This is in contrast to methods producing a more gradual rise (from over 20 minutes to several hours) to the maximal insulin concentration achieved and a prolonged plateau near maximal concentrations. It can also refer to methodologies in which the spike in insulin concentration can be reliably coordinated with the start of a meal. It can also refer to methodologies achieving a maximal glucose elimination rate (GERmax) within about 30-90 minutes, preferably around 45-60 minutes, after administration. A methodology that mimics early phase release is generally also one that can be practiced by diabetics upon themselves without special medical training, such as training in intravenous injection. Special medical training would not include training to use medical devices, such as dry powder inhalers, that are routinely used by persons who are not [trained] medical professionals. As used herein, "meal", "meals", and/or "mealtime", etc. include traditional meals and meal times; however, these also include the ingestion of any sustenance regardless of size and/or timing. Nonetheless it is preferred that insulin be administered only for a meal providing at least a threshold glycemic load depending on the insulin dose so as to avoid a risk of hypoglycemia.

The potentiation of GER contributing to the rapid attainment of GERmax is understood to depend not only on the rapidity of the rise in insulin concentration, but also on achieving sufficient peak height. For type 1 diabetics this is a peak insulin concentration of at least about 60 mU/L, preferably at least about 80 mU/L. For type 2 diabetics the insulin resistance that is part of the condition necessitates higher insulin concentrations; typically at least about 100 mU/L, preferably at least about 120 mU/L, at least about 140 mU/L, or more, depending on the degree of resistance. These peak insulin concentrations are substantially higher than those attained with typical doses of non-spiking insulin products such as standard preparations for subcutaneous administration, including those termed fast-acting, and preparations for non-injected administration having similar kinetics that are now being developed.

It is the applicant's further understanding that a high surge and rapid rate of change in insulin concentration suppresses glucagon production, reducing hepatic gluconeogenesis. This results in lessened glycemic load and consequently lessened demand for insulin and reduced glucose excursion.

The patient populations treated according to the methods herein disclosed are not entirely coincident with those most commonly receiving insulin therapy. Indeed the method can be practiced to great advantage at the earliest stages of these conditions, when functional β-cell populations are greatest, even though current insulin therapies are commonly not offered to these patients. Thus as used herein "a patient in need of insulin therapy" comprises such populations. These patients are also defined as non-insulin dependent patients. Generally, patients with some insulin production capacity, but inadequate early phase release, constitute preferred target populations selected for treatment in embodiments of the invention. Such populations include, without limitation, recipients of transplants of islets, β-cells, or cells engineered to produce insulin, and recipients of insulin gene therapies; type 1 diabetics in the honeymoon phase or in whom the disease is incipient; and type 2 diabetics traditionally treated with diet and exercise, oral medications, long-acting (basal) insulin only, or short-acting insulin - alone or mixed with long-acting insulin - in conjunction with two or fewer daily meals. Such populations include patients with otherwise acceptable HbA1c (glycated hemoglobin) levels (a measure of chronic hyperglycemia) who would not generally be treated by any particular modality, or at all. Normal HbA1c levels are 4.5% to 5.7% (or when reported with less precision 4-6%). Treatment of diabetes generally aims to reduce HbA1c levels to below 7%. HbA1c levels above 8% indicate that patient's current therapy should be re-evaluated. It would be desirable to achieve normal HbA1c levels, but with the currently marketed insulin products this could only be accomplished at an unacceptable risk of severe hypoglycemia. In embodiments of insulin preparations of the present invention the risk of hypoglycemia is much reduced and it is possible to treat patients with HbA1c below 7%. Thus patients with HbA1c levels below 8% would not be considered candidates for more intensive treatment, that is, for treatment with insulin; or if already receiving basal or mixed insulin, for treatment with prandial insulin regimen. Additionally, benefit is expected from lowering blood glucose even at the high end of the normal range, so that patients with HbA1c levels ≤ 6% are selected for treatment. While the invention is generally discussed in reference to human patients adaptation to non-human mammals is not beyond the scope of the invention or the abilities of one of skill in the related arts.

Patients with early stage insulin disorders can be divided into various subpopulations and treated according to various embodiments of the present invention. Some persons make sufficient insulin to maintain a non-hyperglycemic fasting blood glucose level but cannot avoid acute fluctuations in blood glucose after eating. Patients with impaired fasting glucose or impaired glucose tolerance, that is with a fasting blood glucose level between 100 mg/dL (5.5 mmol/L) and 126 mg/dL (7.0 mmol/L), or a 2 hour post-prandial blood glucose level between 146 mg/dL (7.9 mmol/L) and 200 mg/dL (11.1 mmol/L), often termed pre-diabetics, can be treated to delay or prevent progression to diabetes. Early type 2 diabetics can often use diet and exercise to control even substantial hyperglycemia, but will have already lost their early phase insulin release. In current practice patients failing diet and exercise are most often next treated with an insulin sensitizer, such as metformin, with the goal of overcoming insulin resistance and improving the effectiveness of the insulin that is produced. In embodiments of the present invention these patients are administered a prandial, early phase-mimicking insulin preparation instead of, or in addition to, the insulin sensitizer. Less often (and previously) the first oral medication offered diabetics was an insulin secreiagogue, such as a sulfonylurea, to increase insulin secretion. However, increasing insulin secretion may increase metabolic stress on the islets so, a prandial, early phase-mimicking insulin preparation is used instead of a secretagogue.

A deficiency with existing formulations of insulin for subcutaneous injections has been the unpredictable variability of absorption, and the relatively slow rise in serum insulin levels compared to physiologic meal-related early phase insulin response, in which serum insulin levels can peak within about 6 minutes. Meal-related early phase insulin originates from storage vesicles in the β-cells of the islets of Langerhans of the pancreas, where proinsulin undergoes enzymatic cleavage into insulin and C-peptide. The lack of an adequate early phase response is a common element in the earlier phases of type 1 and 2 diabetes and in islet transplant recipients. The rapid release of large amounts of insulin to create the characteristic spike in blood insulin concentration places a significant biosynthetic load on the pancreas. The loss of adequate early phase release is an indicator of a stressed or impaired pancreas, but also contributes to further stressing the β-cells. Diabetes is further characterized by elevated levels of serum proinsulin. Such circulating intact proinsulin (iPi) signifies that insulin requirements exceed β-cell capacity, causing premature release, and reflecting pancreatic stress.

The comparatively slow and shallow rise in insulin concentration and prolonged period of action associated with insulin preparations that do not mimic early phase release limits their ability to control glucose excursions. The dose that can be given is generally inadequate to control the rise in blood glucose following a meal by the need to avoid inducing hypoglycemia after the glycemic load from the meal has been abated. These issues are further discussed in co-pending U.S. Patent Application No. 11/278,381 entitled "Superior Control of Blood Glucose in Diabetes Treatment". It is emerging that acute fluctuations in blood glucose concentrations (measured for example as MAGE: mean amplitude of glycemic excursions) have a greater effect than chronic hyperglycemia (typically measured as Hb1Ac level) on diabetes-associated oxidative stress, and thus is an important parameter to control to avoid diabetic complications attributable to such stress (see Monnier, L., et al. JAMA 295:1681-1687, 2006; and Brownlee, M. & Hirsch, I. JAMA 295:1707-1708).

Insulin therapy has traditionally focused on controlling average blood glucose concentrations, as reflected by Hb1Ac levels. Thus relatively few patients capable of producing significant amounts of insulin receive basal-prandial therapy (involving administration of a basal insulin plus insulin with every meal). More common approaches involve long-acting (basal) insulin alone, mixtures of fast and intermediate acting, and various other combinations of injections. Clinical criteria for the adoption of one or another of these regimens are not well-defined. Generally treatment starts with basal insulin, if or when that is not successful in achieving target Hb1Ac levels, therapy is intensified using additional injections and various mixtures (pre-mixed or self-mixed). If the target is still not achieved, treatment progresses to basal-prandial therapy. Prandial therapy in patients not receiving basal insulin is uncommon and, using the presently marketed (non-spiking) insulin preparations, does not confer the alleviation of pancreatic stress of the instant invention. Moreover currently available insulin preparations provide activity over longer time frames than preferred in the present invention making them less suitable for the control of glucose excursions.

The present invention is designed to minimize not only Hb1Ac levels (average blood glucose concentration) and attendant glucose toxicity; but also to reduce biosynthetic demand for insulin by providing an exogenous insulin mimicking an early phase response, and control acute fluctuations in glucose concentration (glucose excursions) further reducing insulin demand. The reduction of glucose excursions also relieves the general inflammatory burden and oxidative damage to microvasculature resulting from oxidative stress, generally and in the islets. This is accomplished by routinely administering an insulin preparation that mimics early phase release in conjunction with at least three meals a day, preferably with every meal or snack. Such treatment should be maintained, in increasing preference and for increasing effectiveness, for any number of days, weeks, months, and years, up to the remainder of the patient's life or until such time as the underlying insulin-related disorder is cured. It is the non-binding hypothesis of the applicants that under such supportive treatment as described herein, pancreatic function will improve over time, e.g. due to increased β-cell mass, resulting in recovered ability for endogenous early phase release. Under such conditions it may be possible to reduce the number of daily administrations. By routinely it is meant that the advocated schedule of administration is the ideal and usual usage, but in real world practice deviations from this protocol, such as occasional missed doses, do not depart from the scope of the invention. Also disclosed herein, insulin is administered with any meal or snack that would otherwise cause blood glucose to exceed 140 mg/dL; with any meal or snack constituting 1, 2, 3, or more bread exchanges; with any meal or snack containing more than about 15, 20, 30, or 45 g of carbohydrate.

Also disclosed herein is a variety of dosing regimens including, but not limited to, dosing at every meal or snack, dosing at every meal or snack having a carbohydrate content of more than 15 g, dosing at every meal or snack having a carbohydrate content of more than 30 g, every meal or snack having a carbohydrate content of more than 45 g. Dosages and desired insulin composition concentrations may vary depending on the particular use envisioned. The determination of the appropriate dosage or route of administration is well within the skill of an ordinary physician. Furthermore the length of treatment according to the present invention may vary on the particular use and determination of the length of treatment is within the skill of an ordinary physician.

In the case of type 1 diabetes, an initial inflammation and autoimmune attack reduces the number of functional β-cells thereby limiting the biosynthetic capacity of the islets. Similarly, one explanation for islet failure in transplant patients is that they receive too few islets, even if they get cells from multiple donors. A normal pancreas has roughly 1 million islets, but current techniques allow only 400,000 cells at most to be extracted from a donor pancreas and many of these die soon after transplantation. Indeed, islet exhaustion due to chronic overstimulation of a marginal islet cell mass is understood to be the dominant reason for late transplant dysfunction (Shapiro, A.M.J., The Scientist 20:43-48, 2006) In either case, the remaining islet cells are forced to labor unusually hard and can lose function over time. This can be further exacerbated by the absence of the potentiating effect the spike in insulin concentration has on subsequent insulin levels as it will increase the amount of insulin needed in second-phase release to handle the glucose load resulting from a meal. This potentiation effect is more fully described in co-pending U.S. Patent Application No. 11/329,686 filed 1/10/06, entitled "Potentiation of Glucose Elimination." In type 2 diabetes, at least initially, there is not an overt shortage of islet cells, but insulin resistance reduces the effectiveness of the insulin that is produced beyond that due to the loss of potentiation that results when early phase release is lost. This poses a requirement for more insulin to clear a glucose load, again placing stress on the pancreas for insulin production. This inefficiency of glucose elimination also results in prolonged elevations in glucose concentration and consequent glucose toxicity on β-cells. Through these various paths, the biosynthetic capacity of the islets becomes overwhelmed. In this case, there is progressively diminished insulin secretion, subsequent loss of second-phase insulin release and, ultimately, complete insulin deficiency.

As pancreatic stress is believed to be a cause for the progression of type 1 and 2 diabetes and the loss of islet transplant function, so it follows that a method to reduce pancreatic stress would improve the function and lifespan of insulin-producing cells in these patient populations. By using a method of insulin delivery that mimics early phase release many of the deficits caused by it the loss of this response can be alleviated, thereby reducing the attendant stress(es). By creating a spike in insulin concentration at the beginning of a meal the β-cells are relieved of this demand, allowing them to more readily supply other insulin needs (i.e., second-phase and basal insulin). Moreover, the potentiation that results from an early phase-like spike reduces the amount of insulin needed in the second phase release for any particular glucose load even in a background of insulin resistance. The more efficient use of insulin also contributes to reducing the magnitude and duration of any excursions from normal glucose levels in the blood alleviating the effects of glucose toxicity and oxidative stress. Thus, the spiral of increasing biosynthetic demand and decreasing capacity can be interrupted and the requirement for insulin production brought more closely in line with remaining capacity.

In the case of islet transplantation some antirejection drugs, such as sirolimus, inhibit islet revascularization and are understood to interfere with the islet. graft's capacity for regeneration (Shapiro, A.M.J., The Scientist 20:43-48, 2006). Thus an adjunctive insulin therapy that minimizes other causes of vascular damage, such as oxidative stress due to acute fluctuations of blood glucose concentrations, may be particularly advantageous in this context.

Intravenous injection of insulin can effectively replicate the early phase response, but is not a practical therapy for a lifelong condition requiring multiple daily administrations. Traditional subcutaneous injections are absorbed into the bloodstream slowly by comparison, even using fast-acting formulations, which still take up to an hour to reach maximal concentration in the blood and have a plateau lasting several hours. Many pulmonary formulations that have been assessed are equivalent to subcutaneous insulin in effectiveness and similarly fail to achieve the rapid kinetics needed to mimic early phase release, as defined above. Nonetheless, the potential for truly fast absorption using a non-injection based delivery, such as pulmonary and oral administration does exist. For example, pulmonary delivery using diketopiperazine-based dry powder formulations have been utilized.

The loss of early phase insulin response, increased proinsulin levels, and decreased glucose control in a diabetic patient are each a measure of loss of function of insulin-producing cells. This loss of function can be attributed to cell death and/or islet cell stress. In fact, the greatest load placed on insulin producing cells in diabetics is to release insulin for the early phase response. Administration of an exogenous source of insulin to mimic this response can eliminate this load (stress) and preserve basal and second-phase (meal related, glucose-dependent) insulin release.

Also disclosed herein is a method to achieve the desirable early phase kinetics through pulmonary administration of a dry powder insulin formulation containing insulin complexed to diketopiperazine microparticles. This formulation is rapidly absorbed reaching peak serum levels within about 10 to 15 minutes. This is fast enough to mimic the kinetics of the physiologic meal-related early phase insulin response. The short, sharp rise to peak serum insulin concentration is critical to relieving the biosynthetic demand otherwise placed upon the β-cells and has the additional effect of compressing the bulk of insulin action to the peri-prandial time interval, in contrast with slower acting formulations. This reduces the magnitude and duration of any meal-related excursions from normal glucose levels and associated glucose toxicity, as well as the risk of post-prandial hypoglycemia. Such improved control of blood glucose levels obtainable with this dry powder insulin is more fully described in co-pending U.S. Patent Application No. 11/278,381, filed 3/31/06, entitled "Superior Control of Blood Glucose Levels in Diabetes Treatment". As disclosed in U.S. Appl. No. 11/329,686 and noted above, prior high insulin levels potentiate glucose elimination rate, meaning glucose can be eliminated more quickly if there is a prior high insulin concentration spike. Such treatment also leads to reduced levels of serum proinsulin, indicating a reduction of biosynthetic pancreatic stress.

Diketopiperazine microparticle drug delivery systems and associated methods are described in U.S. Patents 5,352,461 and 5,503,852 entitled "Self Assembling Diketopiperazine Drug Delivery System," and "Method for Making Self Assembling Diketopiperazine Drug Delivery System," respectively. The use of diketopiperazine and biodegradable polymer microparticles in pulmonary delivery is described in U.S. Patents 6,428,771 and 6,071,497 entitled "Method for Drug Delivery to the Pulmonary System," and "Microparticles for Lung Delivery Comprising Diketopiperazine," respectively. Details regarding various aspects of possible formulation and manufacturing processes can be found in U.S. Patents 6,444,226 and 6,652,885 both entitled "Purification and Stabilization of Peptide and Protein Pharmaceutical Agents"; in U.S. Patent 6,440,463 entitled "Methods for Fine Powder Formation"; in co-pending U.S. Provisional Patent Application Nos. 60/717,524, filed 9/14/05, entitled "Method of Drug Formulation Based on Increasing the Affinity of Active Agents for Crystalline Microparticle Surfaces"; and 60/776,605, filed 4/14/06, entitled "A Method for Improving the Pharmaceutic Properties of Microparticles Comprising Diketopiperazine and an Active Agent". The properties and design of a preferred breath-powered dry powder inhaler system is disclosed in U.S. Patent Application No. 10/655,153 entitled "Unit Dose Cartridge and Dry Powder Inhaler." Aspects of treatment using insulin complexed to diketopiperazine microparticles are disclosed in U.S. Patent 6,652,885 as well as in co-pending U.S. Patent Application No. 11/032,278, entitled "A Method of Reducing Serum Proinsulin Levels in Type 2 Diabetes.." Additionally U.S. Patent Application No.11/210,710 entitled "Diketopiperazine Salts for Drug Delivery and Related Methods" discloses the use of diketopiperazine salts to formulate insulin for both pulmonary and oral delivery.

Also contemplated, any medications may be administered in combination with the insulin disclosed herein. These medications may include, but are not limited to oral antidiabetic medications, incretin mimetics, those that preserve β-cell function, co-stimulation blockading agents such as anti-CD28 antibodies or belatacept, anti-CD3_ antibodies, and/or any immunosuppresive medication (typically used in an islet transplant case), however, medications that preserve β-cell function are preferred. Exemplary immunosuppressive medications include, but are not limited to, daclizumab, sirolimus, tacrolimus, mycophenolic acid, rapamycin, glucocorticoids, prenisone, azathioprine, and cyclosporine however, glucocorticoids, prenisone, azathioprine, and cyclosporine are among those that are less preferred.

More specifically, herein is disclosed administering TECHNOSPHERE^{®}/Insulin (TI) in conjunction with an immunosuppressive medication(s), for example, an anti-CD3 antibody, to prolong the honeymoon phase in type 1 diabetics. These anti-CD3 antibodies block the function of immune T cells, which are the cells responsible for the destruction of the beta islet cells in the pancreas. The term "in conjunction" as used herein means that the TI and immunosuppressive medication(s) are used as dual therapies to ultimately reduce pancreatic stress.

Whether TI or another insulin mimicking early phase release is administered alone or in conjunction with an immunosuppressive medication, the insulin may be administered in association with meals, preferably one to four times daily, depending upon need. In order to achieve the maximum benefit of the treatment, it should be taken over an extended period of time, preferably up to about one month, more preferably from about two months to about six months, and most preferably for the remaining life of the patient or until the underlying diabetes is cured. One indicator of effectiveness and/or a monitoring parameter includes a periodic assessment of the patient's proinsulin levels. Of course, the frequency of administration and the dosage amount may be adjusted according to this periodic proinsulin determination:

Also disclosed herein is a method of treating diabetic patients with an amount of pulmonary administered dose of dry powder TI sufficient to mimic early phase insulin response, to lower serum proinsulin levels, and/or to control blood glucose levels in order to improve the longevity of the insulin producing cells in early type 1 and 2 diabetics and islet transplant patients, as described in the examples below.

### EXAMPLES

### Example 1

### A Randomized, Double-Blind, Placebo Controlled Study of the Efficacy and Safety of Inhaled TECHNOSPHERE^{®}/Insulin in Patients with Type 2 Diabetes

TECHNOSPHERE^{®} dry powder, pulmonary insulin delivered via a small pulmonary inhaler has a bioavailability that mimics normal, meal-related, first- or early-phase insulin release. This multicenter, randomized, double-blind, placebo-controlled study was conducted in type 2 diabetes mellitus patients inadequately controlled on diet or oral agent therapy (HbA1c >6.5% to 10.5%). A total of 123 patients were enrolled and 119, the intention-to-treat population (ITT), were randomized in a 1:1 ration to receive prandial inhaled TECHNOSPHERE^{®}/Insulin from unit dose cartridges containing between 6 to 48 units of human insulin (rDNA origin) or inhaled TECHNOSPHERE^{®}/placebo (PBO).

Glycosylated hemoglobin A1c (HbA1c) results were analyzed by a predetermined statistical analysis plan for the Primary Efficacy Population (PEP, defined prior to un-blinding as those who adhered to study requirements including minimal dosing and no adjustments of concomitant diabetes drugs), for a PEP Sub-group A (those with baseline HbA1c of 6.6 to 7.9%), for a PEP Sub-group B (those with baseline HbA1c of 8.0 to 10.5%), as well as for the ITT. These results are summarized in Table 1. In this individualized dose study, the mean dose of TI used before each meal in the active treatment group was approximately 30 units, with 28 units used in PEP Sub-group A and 33.5 units used in PEP Sub-group B.

**Table 1**

| | **TECHNOSPHERE^{®}/Placebo** | **TECHNOSPHERE^{®}/Insulin** |
|---|---|---|
| PEP n=90 | n=42 | n=48 |
| Mean HbA1c Baseline (%) | 7.75 | 7.74 |
| Mean Δ from baseline | -0.32 | -0.76 (p<0.0001) |
| Comparison to Placebo | | p=0.0019 |
| | | |
| PEP Sub-group B n=35 | n=18 | n=17 |
| Mean HbA1c Baseline (%) | 8.52 | 8.72 |
| Mean Δ from baseline | -0.51 (p=0.0094) | -1.37 (p<0.0001) |
| Comparison to Placebo | | p=0.0007 |
| | | |
| PEP Sub-group A n=35 | n=24 | n=31 |
| Mean HbA1c Baseline (%) | 7.16 | 7.19 |
| Mean Δ from baseline | -0.18 (p=0.1292) | -0.43 (p=0.0001) |
| Comparison to Placebo | | p<0.05 |
| | | |
| IIT (LOCF) n=119 | n=61 | n=58 |
| Mean HbA1c Baseline (%) | 7.78 | 7.87 |
| Mean Δ from baseline | -0.31 (p=0.0020) | -0.72 (p<0.0001) |
| Comparison to Placebo | | p=0.0016 |

No episodes of severe hypoglycemia occurred in the TI group. Pulmonary function tests, including Dlco, FEV1, and Total Alveolar Volume showed no significant differences between patients on TI compared to their baseline values or compared to the results of those receiving PBO. There was no evidence of induction of insulin antibodies with TI during the 12 week period of exposure.

### Example 2

### Mimicry of the Early Phase Insulin Response in Humans with Rapidly Bioavailable Inhaled Insulin Accelerates Post Prandial Glucose Disposal Compared to Insulin with Slower Bioavailability

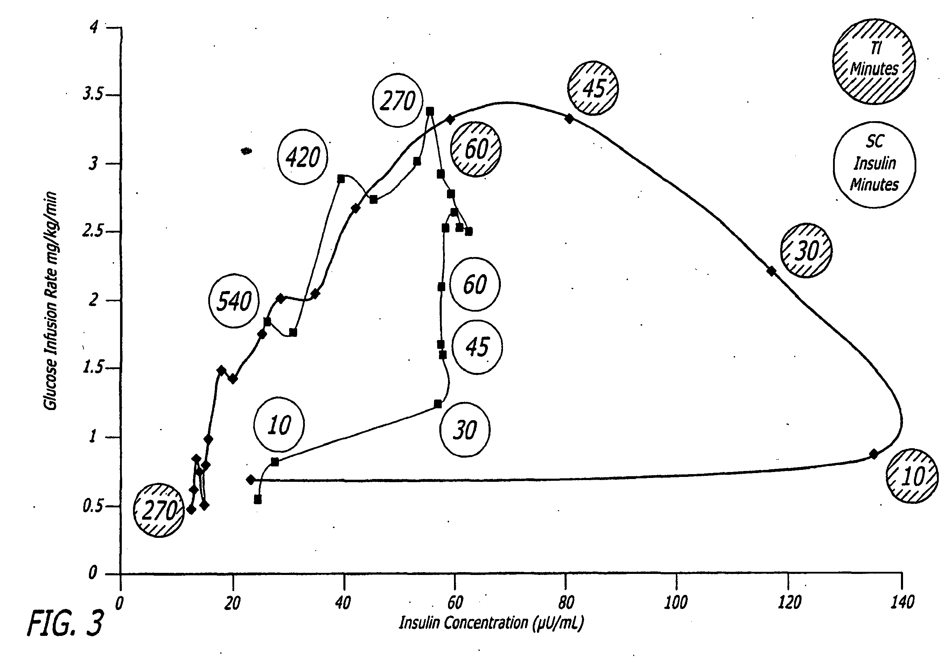

The relationship between time, insulin concentration, and glucose elimination rate in a group of 12 subjects with type 2 diabetes, during an isoglycemic clamp was studied. Each subject received 24 IU (International Units) subcutaneous insulin (Actrapid^{®}, Novo Nordisk) or 48 U TECHNOSPHERE^{®}/Insulin (TI, MannKind Corporation)) on separate study days in a cross-over design. Glucose Elimination Rate (GIR) was determined by the amount of glucose infusion required to maintain stable blood glucose of 120 mg/dL during the 540 minute study period (Figure 3).

Forty-eight units TI provided a mean maximum concentration of insulin (Cmax) of 114.8 ± 44.1 (mean ± SD) mU/L and had a median time to maximum concentration (Tmax) of 15 min, whereas 24 IU subcutaneous insulin (SC) had a Cmax of 63 ± 10.1 mU/L with a Tmax of 150 min. TECHNOSPHERE^{®}/Insulin reached maximal GIR values, 3.33 ± 1.35 mg/min/kg, at 45 min, while at that time point, SC was only 1.58 ± 1.03 and did not reach maximal value, 3.38 + 1.45 before 255 min, despite almost constant insulin concentrations. Once maximal insulin effect was reached, the concentration-effect relationship was the same for TI and SC. At 180 min, glucose disposal was 326 ± 119 mg/kg or 61% of total for TI and 330 ± 153 mg/kg (27% of total) for SC.

A fast, sharp increase in insulin concentration, similar to the early phase insulin response, provide maximal glucose elimination rate. Forty-eight units TI achieved maximal effect within 45 min, whereas it took 270 min for 34 IU SC to reach similar effect. This phenomenon is not caused by differences in the dose-effect relationship for the two insulin types, but reflects a difference in response when the increment in insulin concentration is more modest over time as opposed to the more rapid bioavailable insulin provided by TECHNOSPHERE^{®}/Insulin. This can have consequences for post prandial glucose control.

Also, three hours after dosing, 48 U TI and 24 IU SC had exerted the same glucose lowering effect. However, less than 1/3 of the total glucose lowering effect for the SC dose had been obtained. If the prandial insulin dose is titrated towards a goal of normoglycemia at three hours after a meal, the large remaining glucose lowering effect of SC insulin may increase the risk of late post prandial hypoglycemia, as compared to TI.

One problem with existing formulations of insulin for subcutaneous injections has been the unpredictable variability of absorption and the relatively slow rise in serum insulin levels compared to physiologic meal-related first-phase insulin response, in which serum insulin levels can peak within about six minutes. Therefore, the preferred kinetics of insulin formulations for prandial substitution includes a rapid and early onset of action and a duration of action long enough to cover meal-related glucose absorption. Pulmonary TECHNOSPHERE^{®}/Insulin meets this requirement by mimicking early phase insulin response in diabetic patients who have lost this function. Islet transplant patients represent a population of treated diabetic patients that still do not exhibit first-phase insulin response. Administration of TECHNOSPHERE^{®}/Insulin to islet transplant patients restores first phase-like insulin response thereby reducing pancreatic stress and improving the longevity of the transplanted cells.

### Example 3

### Treatment of Humans with Pulmonary Insulin Reduces Serum Proinsulin Levels

Inhalation of TECHNOSPHERE^{®}/Insulin (TI) provides a rise in serum insulin, comparable to the first phase response. This study investigated the pharmacodynamics of TI and its impact on intact proinsulin (iPi) release. Twenty-four patients with Type 2 diabetes received doses of TECHNOSPHERE^{®} base with 4 different loadings of insulin, either 0, 12 IU, 24 IU, or 48 IU of recombinant regular human insulin, five minutes after the start of standardized meals, on separate study days. Blood glucose (BG), serum insulin and serum iPi were measured before (0 min), 60 and 120 min after initiation of each meal.

TI lowered postprandial BG levels in a dose-dependent manner. Sixty minutes after lunch, BG (mg/dL) (± SD) was 183.2 (± 44.4) for placebo; 170.8 (± 30.5) for 12 IU (p=0.266); 156.3 (± 31.9) for 24 IU, (p=0.020) and 132.6 (± 29.1) for 48 IU, (p<0.001). All doses caused an increase in serum insulin at 60 minutes (p<0.05), but not at 120 minutes following inhalation. Administration of TI with 24 IU and 48 IU insulin load doses suppressed iPi levels at all time points throughout the day (p<0.05). The use of inhaled TI to mimic the rapid onset and short duration of the first phase insulin response therefore should reduce I stress on insulin producing cells. This can improve general β-cell function, endogenous glucose homeostasis, and the longevity of residual and transplanted β-cells.

Figure 3 depicts the changes in proinsulin levels over time, following pulmonary administration of diketopiperazine/insulin particles.

### Example 4

### Evaluation of β-cells in Diabetic Fatty Rats Treated with Pulmonary Insulin

Diabetic Fatty Rats are a model of type 2 diabetes. Two strains, ZDF and WDF, are available. Diabetes can be induced in the WDF strain by feeding a high sucrose diet for approximately one week. Alternatively the ZDF rats will develop diabetes spontaneously at about 13 weeks of age.

Three groups of 20 rats are treated with daily doses of either insulin by subcutaneous injection, TI by pulmonary insufflation, or air by pulmonary insufflation. Dosing commences one week prior to anticipated onset of diabetes. Dosage is selected to approximate an equivalent of a human dose, but less than would cause severe or life-threatening hypoglycemia in the not yet diabetic animals. The animals are fasted overnight prior to taking blood measurements, but are otherwise fed *ad libitum.*

Body weights are measured weekly. Serum blood glucose measurements are conducted twice per week. Glycosuria testing is conducted three times per week. Levels of glucose in the urine greater than 250 mg/dL are followed by glucometer test for 2 days to confirm diabetes onset. Upon confirmation of diabetes onset, animals are sacrificed within 24-48 hours. Insulin, intact proinsulin, and C-peptide are measured from a pre-dose blood sample, weekly in-life blood samples during dosing, and in the terminal blood sample. The pre-dose and in-life samples are taken as pairs just before and 3-5 minutes after bolus glucose challenge so that first phase insulin release can be assessed. At sacrifice, pancreases from all animals are harvested and fixed in 10% formalin. Pancreatic tissue is processed for hemotoxylin and eosin (H & E) staining and evaluated for β-cell mass. Proliferation and apoptotic indices are also evaluated by immunohistochemistry (IHC) in pancreatic tissues.

Reduced stress and prolonged β-cell longevity in the TI group is indicated by greater β-cell mass, greater expression of proliferation markers lower apoptotic index, delayed progression to and onset of diabetes. Progression to diabetes is assessed from the rise over time of levels of blood glucose, insulin, and C-peptide; absence or lower levels of intact serum proinsulin; and delayed loss of first-phase insulin release.

### Example 5

### Evaluation of β-cells in NOD mice Treated with Pulmonary Insulin

NOD (non-obese diabetic) mice are a model of type 1 diabetes. Diabetes develops spontaneously at about 12-14 weeks of age, with the variance being less in females than in males.

Three groups of 40 female mice are treated with daily doses of either insulin by subcutaneous injection, TI by inhalation, or air by inhalation. Dosing commences, prior to anticipated onset of diabetes, at 10 weeks of age. Dosage is selected to approximate an equivalent of a human dose, but less than would cause severe or life-threatening hypoglycemia in the not yet diabetic animals. The animals are fasted overnight prior to taking blood measurements, but are otherwise fed *ad libitum.*

Body weights are measured weekly. Serum blood glucose measurements are conducted twice per week. Glycosuria testing is conducted three times per week. Levels of glucose in the urine greater than 250 mg/dL are followed by glucometer test for 2 days to confirm diabetes onset. Upon confirmation of diabetes onset, animals are sacrificed within 24-48 hours. Insulin and C-peptide are measured from a pre-dose blood sample, two equally spaced in-life blood samples during dosing, and the terminal blood sample. The pre-dose and in-life samples are taken as pairs just before and 3-5 minutes after bolus glucose challenge so that first phase insulin release can be assessed. At sacrifice, pancreases from all animals are harvested and fixed in 10% formalin. Pancreatic tissue is processed for H & E staining and evaluated for β-cell mass. Proliferation and apoptotic indices are also evaluated by IHC in pancreatic tissues.

Reduced stress and prolonged β-cell longevity in the TI group is indicated by greater β-cell mass, greater expression of proliferation markers, lower apoptotic index, delayed progression to and onset of diabetes. Progression to diabetes is assessed from the rise over time of levels of blood glucose, and fall of insulin and C-peptide; and delayed loss of first-phase insulin release.

## Claims

1. An insulin composition for use in the preservation of insulin-producing cells in a patient with type 1 diabetes in the honeymoon phase or in a patient with type 1 diabetes who is a recipient of an insulin-producing cell transplant wherein at least one dose of said composition is administered prandially to said patient and wherein said composition mimics a physiological meal-related early phase insulin response, such that serum insulin levels peak within 15 minutes of administration, and preserves the function of said insulin-producing cells.

2. The composition for use according to claim 1, wherein said composition is administered orally.

3. The composition for use according to claim 1, wherein said composition is inhaled.

4. The composition for use according to claim 3, wherein said composition comprises a dry powder formulation.

5. The composition for use according to claim 1 wherein said composition is administered at any meal containing more than 15 g of carbohydrate.

6. The composition for use according to claim 1, wherein said composition comprises an insulin dose sufficient to reduce serum levels of proinsulin.

7. The composition for use according to claim 1 wherein said composition comprises an insulin dose sufficient to control glucose excursions.

8. The composition for use according to either of claims 6 or 7, wherein said dose is sufficient to control blood glucose levels.

9. The composition for use according to either of claims 6 or 7, wherein said dose is sufficient to reduce glucose release from the liver.

10. The composition for use according to claim 1 wherein said peak serum insulin level is at least 60 mUIL.

11. The composition for use according to claim 1 wherein said insulin composition comprises a fumaryl diketopiperazine (FDKP) associated with insulin.

12. The composition for use according to claim 11, wherein said dose is within the range equivalent to 15 IU to 90 IU of FDKP insulin.

13. The composition for use according to claim 1 wherein said patient is further treated with an insulin sensitizer or an insulin secretagogue.

14. The composition for use according to claim 1 wherein said dose of the composition is sufficient to control blood glucose levels and reduce serum levels of proinsulin, and whereby pancreatic stress is attenuated.

15. The composition for use according to claim 1 wherein said dose of the composition being sufficient to control blood glucose levels and reduce serum levels of proinsulin, whereby pancreatic stress is attenuated and longevity of insulin-producing cells is achieved.

16. The composition for use according to claim 1 wherein said patient is administered at least one dose of said composition and an immunosuppressive medication, said composition is administered prandially and wherein said composition and said immunosuppressive medication slow an auto-immune response.

17. The composition for use according to claim 1 comprising a controlled-release insulin formulation.

18. The composition for use according to claim 1 comprising a delayed onset preparation including an insulin formulation.

19. The composition for use according to claim 1 wherein said patient has lost early phase insulin release and has a level of serum glycated hemoglobin (HbA1c) less than 8%.

20. The composition for use according to claim 19, wherein said composition is administered with any meal containing more than 15 g of carbohydrate.

21. The composition for use according to claim 19, wherein said patient is not on a prandial insulin regimen.

22. The composition for use according to claim 19, wherein said patient has elevated serum proinsulin levels.

23. The composition for use according to claim 19, wherein said patient has elevated mean amplitude of glucose excursions.

24. The composition for use according to claim 19, wherein said patient has evidence of elevated oxidative stress.

25. The composition for use according to claim 19, wherein said level of serum HbA1c is less than 7%.

26. The composition for use according to claim 19, wherein said level of serum HbA1c is less than 6.5%.

27. Use of an insulin composition in the manufacture of a medicament for the preservation of insulin-producing cells in a patient with type 1 diabetes in the honeymoon phase or in a patient with type 1 diabetes who is a recipient of an insulin-producing cell transplant wherein at least one dose of said composition is administered prandially to said patient and wherein said composition mimics a physiological meal-related early phase insulin response, such that serum insulin levels peak within 15 minutes of administration, and preserves the function of said insulin-producing cells.

## Patentansprüche

1. Insulinzusammensetzung zur Verwendung bei der Erhaltung von Insulin-produzierenden Zellen bei einem Patienten mit Diabetes Typ 1 in der Honeymoon-Phase oder bei einem Patienten mit Diabetes Typ 1, der ein Empfänger eines Insulin-produzierenden Zelltransplantats ist, wobei mindestens eine Dosis der Zusammensetzung prandial an den Patienten verabreicht wird und wobei die Zusammensetzung eine physiologische, Mahlzeiten-bezogene Frühphasen-Insulinantwort nachahmt, derart, dass die Insulinspiegel im Serum innerhalb von 15 Minuten nach der Verabreichung einen Peak erreichen, und die Funktion der Insulin-produzierenden Zellen aufrecht erhält.

2. Zusammensetzung zur Verwendung gemäß Anspruch 1, wobei die Zusammensetzung oral verabreicht wird.

3. Zusammensetzung zur Verwendung gemäß Anspruch 1, wobei die Zusammensetzung inhaliert wird.

4. Zusammensetzung zur Verwendung gemäß Anspruch 3, wobei die Zusammensetzung eine Trockenpulverformulierung umfasst.

5. Zusammensetzung zur Verwendung gemäß Anspruch 1, wobei die Zusammensetzung bei jeder Mahlzeit, die mehr als 15 g Kohlenhydrate enthält, verabreicht wird.

6. Zusammensetzung zur Verwendung gemäß Anspruch 1, wobei die Zusammensetzung eine zum Verringern der Serumspiegel von Proinsulin ausreichende Insulindosis umfasst.

7. Zusammensetzung zur Verwendung gemäß Anspruch 1, wobei die Zusammensetzung eine zur Steuerung von Glucoseabweichungen ausreichende Insulindosis umfasst.

8. Zusammensetzung zur Verwendung gemäß einem der Ansprüche 6 oder 7, wobei die Dosis zum Steuern der Blutglucosespiegel ausreichend ist.

9. Zusammensetzung zur Verwendung gemäß einem der Ansprüche 6 oder 7, wobei die Dosis zum Verringern der Glucosefreisetzung aus der Leber ausreichend ist.

10. Zusammensetzung zur Verwendung gemäß Anspruch 1, wobei der Peakwert des Serum-Insulinspiegels mindestens 60 mU/L beträgt.

11. Zusammensetzung zur Verwendung gemäß Anspruch 1, wobei die Insulinzusammensetzung ein Fumaryl-diketopiperazin (FDKP) in Verbindung mit Insulin umfasst.

12. Zusammensetzung zur Verwendung gemäß Anspruch 11, wobei die Dosis innerhalb des Bereichs liegt, der zu 15 IU bis 90 IU von FDKP-Insulin äquivalent ist.

13. Zusammensetzung zur Verwendung gemäß Anspruch 1, wobei der Patient des Weiteren mit einem Insulin-Sensitizer oder einem Insulin-Sekretagogum behandelt wird.

14. Zusammensetzung zur Verwendung gemäß Anspruch 1, wobei die Dosis der Zusammensetzung ausreicht, die Blutglucosespiegel zu steuern und die Serumspiegel von Proinsulin zu verringern, und wodurch der pankreatische Stress verringert wird.

15. Zusammensetzung zur Verwendung gemäß Anspruch 1, wobei die Dosis der Zusammensetzung zum Steuern der Blutglucosespiegel und zum Verringern der Serumspiegel von Proinsulin ausreichend ist, wodurch der pankreatische Stress verringert wird und die Langlebigkeit von Insulin-produzierenden Zellen erreicht wird.

16. Zusammensetzung zur Verwendung gemäß Anspruch 1, wobei dem Patienten mindestens eine Dosis der Zusammensetzung und eine immunsuppressive Medikation verabreicht werden, wobei die Zusammensetzung prandial verabreicht wird und wobei die Zusammensetzung und die immunsuppressive Medikation eine Autoimmunreaktion verlangsamen.

17. Zusammensetzung zur Verwendung gemäß Anspruch 1, umfassend eine für eine gesteuerte Freisetzung sorgende Insulinformulierung.

18. Zusammensetzung zur Verwendung gemäß Anspruch 1, umfassend eine für einen verzögerten Krankheitsbeginn sorgende Zubereitung, die eine Isulinformulierung umfasst.

19. Zusammensetzung zur Verwendung gemäß Anspruch 1, wobei der Patient eine Frühphasen-Insulinfreisetzung verloren hat und einen Spiegel von Glycohämoglobin (HbA1c) im Serum von weniger als 8 % aufweist.

20. Zusammensetzung zur Verwendung gemäß Anspruch 19, wobei die Zusammensetzung mit jeder Mahlzeit, die mehr als 15 g Kohlenhydrate enthält, verabreicht wird.

21. Zusammensetzung zur Verwendung gemäß Anspruch 19, wobei der Patient nicht unter einem prandiales Insulin umfassenden Therapieschema steht.

22. Zusammensetzung zur Verwendung gemäß Anspruch 19, wobei der Patient erhöhte Proinsulinspiegel im Serum aufweist.

23. Zusammensetzung zur Verwendung gemäß Anspruch 19, wobei der Patient eine erhöhte Mittelwert-Amplitude der Glucoseabweichungen aufweist.

24. Zusammensetzung zur Verwendung gemäß Anspruch 19, wobei der Patient den Befund eines erhöhten oxidativen Stresses zeigt.

25. Zusammensetzung zur Verwendung gemäß Anspruch 19, wobei der Spiegel von Serum-HbA1c niedriger als 7 % ist.

26. Zusammensetzung zur Verwendung gemäß Anspruch 19, wobei der Spiegel von Serum-HbA1c niedriger als 6,5 % ist.

27. Verwendung einer Insulinzusammensetzung bei der Herstellung eines Medikaments zur Erhaltung von Insulin-produzierenden Zellen bei einem Patienten mit Diabetes Typ 1 in der Honeymoon-Phase oder bei einem Patienten mit Diabetes Typ 1, der ein Empfänger eines Insulin-produzierenden Zelltransplantats ist, wobei mindestens eine Dosis der Zusammensetzung prandial an den Patienten verabreicht wird und wobei die Zusammensetzung eine physiologische, Mahlzeiten-verbundene Frühphasen-Insulinantwort nachahmt, derart, dass die Serum-Insulinspiegel innerhalb von 15 Minuten nach der Verabreichung einen Peak erreichen, und die Funktion der Insulin-produzierenden Zellen aufrecht erhält.

## Revendications

1. Composition d'insuline pour utilisation dans la préservation de cellules produisant de l'insuline chez un patient atteint de diabète de type 1 lors de la phase de lune de miel ou chez un patient atteint du diabète de type 1 qui est un receveur d'un transplant de cellules produisant de l'insuline, dans laquelle au moins une dose de ladite composition est administrée lors des repas audit patient et dans laquelle ladite composition imite une réponse insulinémique de phase précoce liée au repas, de sorte que les taux sériques d'insuline atteignent un maximum dans les 15 minutes qui suivent l'administration, et préserve la fonction desdites cellules produisant de l'insuline.

2. Composition pour utilisation selon la revendication 1, dans laquelle ladite composition est administrée par voie orale.

3. Composition pour utilisation selon la revendication 1, dans laquelle ladite composition est inhalée.

4. Composition pour utilisation selon la revendication 3, dans laquelle ladite composition comprend une formulation de poudre sèche.

5. Composition pour utilisation selon la revendication 1, dans laquelle ladite composition est administrée à n'importe quel repas contenant plus de 15 g de glucide.

6. Composition pour utilisation selon la revendication 1, dans laquelle ladite composition comprend une dose d'insuline suffisante pour réduire les taux sériques de proinsuline.

7. Composition pour utilisation selon la revendication 1, dans laquelle ladite composition comprend une dose d'insuline suffisante pour réguler les excursions glycémiques.

8. Composition pour utilisation selon l'une ou l'autre des revendications 6 ou 7, dans laquelle ladite dose est suffisante pour réguler les taux de glycémie.

9. Composition pour utilisation selon l'une ou l'autre des revendications 6 ou 7, dans laquelle ladite dose est suffisante pour réduire la libération du glucose à partir du foie.

10. Composition pour utilisation selon la revendication 1, dans laquelle ledit taux sérique d'insuline maximal est d'au moins 60 mUIL.

11. Composition pour utilisation selon la revendication 1, dans laquelle ladite composition d'insuline comprend une fumaryl dicétopipérazine (FDKP) associée à l'insuline.

12. Composition pour utilisation selon la revendication 11, dans laquelle ladite dose se situe dans la plage équivalente à 15 UI à 90 UI d'insuline FDKP.

13. Composition pour utilisation selon la revendication 1, dans laquelle ledit patient est en outre traité avec un insulinosensibilisateur ou un sécrétagogue de l'insuline.

14. Composition pour utilisation selon la revendication 1, dans laquelle ladite dose de la composition est suffisante pour réguler les taux sanguins de glucose et réduire les taux sériques de proinsuline, et grâce à laquelle le stress pancréatique est atténué.

15. Composition pour utilisation selon la revendication 1, dans laquelle ladite dose de la composition est suffisante pour réguler les taux sanguins de glucose et réduire les taux sériques de proinsuline, et grâce à laquelle ledit stress pancréatique est atténué et la longévité des cellules produisant de l'insuline est atteinte.

16. Composition pour utilisation selon la revendication 1, dans laquelle ledit patient se voit administrer au moins une dose de ladite composition et un immunosuppresseur, ladite composition est administrée lors des repas et dans laquelle ladite composition et ledit immunosuppresseur ralentissent une réponse auto-immune.

17. Composition pour utilisation selon la revendication 1, comprenant une formulation d'insuline à libération contrôlée.

18. Composition pour utilisation selon la revendication 1, comprenant une préparation à retardement incluant une formulation d'insuline.

19. Composition pour utilisation selon la revendication 1, dans laquelle ledit patient a perdu la libération d'insuline de phase précoce et a un taux d'hémoglobine glyquée (HbA1c) sérique inférieur à 8 %.

20. Composition pour utilisation selon la revendication 19, dans laquelle ladite composition est administrée à n'importe quel repas contenant plus de 15 g de glucide.

21. Composition pour utilisation selon la revendication 19, dans laquelle ledit patient ne suit pas un régime d'insuline prandiale.

22. Composition pour utilisation selon la revendication 19, dans laquelle ledit patient a des taux sériques de proinsuline élevés.

23. Composition pour utilisation selon la revendication 19, dans laquelle ledit patient a une amplitude moyenne élevée des excursions glycémiques.

24. Composition pour utilisation selon la revendication 19, dans laquelle ledit patient manifeste un stress oxydatif élevé.

25. Composition pour utilisation selon la revendication 19, dans laquelle ledit taux de HbA1c sérique est inférieur à 7 %.

26. Composition pour utilisation selon la revendication 19, dans laquelle ledit taux de HbA1c sérique est inférieur à 6,5 %.

27. Utilisation d'une composition d'insuline dans la fabrication d'un médicament destiné à la préservation des cellules produisant de l'insuline chez un patient atteint de diabète de type 1 lors de la phase de lune de miel ou chez un patient atteint du diabète de type 1 qui est un receveur d'un transplant de cellules produisant de l'insuline, dans laquelle au moins une dose de ladite composition est administrée lors des repas audit patient et dans laquelle ladite composition imite une réponse insulinémique de phase précoce liée au repas, de sorte que les taux sériques d'insuline atteignent un maximum dans les 15 minutes qui suivent l'administration, et préserve la fonction desdites cellules produisant de l'insuline.
